# EUROPEAN PATENT SPECIFICATION

(11) **EP 2 110 146 B1**
(45) Date of publication and mention of the grant of the patent: **24.10.2012**
(21) Application number: 09251086.6
(22) Date of filing: 14.04.2009
(51) Int. Cl.: A61L 17/04, A61L 31/06

(54) **Poly(trimethylene) terephthalate filaments and articles made therefrom**
Poly(trimethylen)-terephthalat-Filamente und Artikel daraus
Filaments de poly(triméthylène) téréphtalate et articles correspondants

(30) Priority: 17.04.2008 US 45755 P; 03.04.2009 US 418086
(43) Date of publication of application: 21.10.2009
(73) Proprietor: Tyco Healthcare Group LP, North Haven, CT 06473 (US)
(72) Inventor: Hotter, Joseph, Middletown, CT 06457 (US); Thomas, Jonathan, New Haven, CT 06511 (US)
(74) Representative: Soames, Candida Jane

(56) References cited:
- WO-A2-2006/138300
- US-A- 2 465 319
- US-A1- 2005 125 036
- US-A1- 2008 021 501

## Description

### CROSS-REFERENCE TO RELATED APPLICATION

The present application claims the benefit of and priority to U.S. Provisional Application Ser. No. 61/045,755, filed on April 17, 2008.

### Technical Field

The present disclosure relates to poly(trimethylene)terephthalate filaments and surgical or medical devices made from such filaments.

### Background

Sutures intended for the repair of body tissues have been constructed from a wide variety of materials. Sutures have been constructed from these materials, for example, in monofilament form or as multifilament structures. Filaments have also been utilized to form other surgical or medical devices, such as braided tapes, gauze, wound dressings, hernial repair meshes, vascular grafts (e.g. fabrics and/or tubes) anastomosis rings, prosthetic ligaments and tendons, growth matrices, drug delivery devices and other implantable medical devices.

WO 2006/138300 A2 discloses bioswellable sutures comprising an absorbable monofilament core and a non-absorbable multifilament braided sheath, the sheath comprising a heterochain polymer, such as polytrimethylene terephthalate.

US 2005/125036 A1 discloses surgical devices made from heterogeneous yarns containing strands made from a polyester and strands made from a polyolefin. In particularly useful embodiments the polyester strands are made from polyethylene terephthalate, and the polyolefin strands are made from an ultra high molecular weight polyethylene.

While many of the existing filamentous surgical and medical devices perform satisfactorily, there remains room for improvement with respect to performance of such articles.

### Summary

Poly(trimethylene)terephthalate filaments, yarns including such filaments and surgical or medical devices made from such filaments are described. In embodiments, the poly(trimethylene)terephthalate filaments are used as monofilament sutures, such as, for example, in sterile needle-suture combinations having a poly(trimethylene)terephthalate homopolymer monofilament secured to a needle. Multifilament sutures including at least one poly(trimethylene)terephthalate filament are also described.

Yarns in accordance with the present disclosure contain at least one filament made from poly(trimethylene)terephthalate. In the present invention, the yarns are heterogeneous yarns that contain one or more poly(trimethylene)terephthalate filaments and one or more filaments made from a fiber forming material. Such heterogeneous yarns can be braided, knitted or woven to form medical/surgical devices, including, but not limited to multifilament sutures. The heterogeneous yarns alternatively can be braided into a surgical article such as a tape, or may be knitted or woven into a mesh. It is also contemplated that non-woven structures such as felt can be made to include fibers of poly(trimethylene)terephthalate.

In embodiments, methods for approximating two tissue surfaces are contemplated. In one embodiment, a method of closing a wound in tissue includes the steps of passing a suture through tissue and securing the ends of the suture to approximate the tissue, wherein the suture is made from at least one poly(trimethylene) terephthalate filament.

In another embodiment, a method of securing soft tissue to hard tissue includes providing a surgical device fabricated from first yarns and second yarns in a braided construction wherein the first yarns include at least one poly(timethylene)terephthalate filament and the second yarns include a plurality of filaments of at least one fiber forming material; passing said surgical device through the soft tissue; securing said surgical device to the hard tissue; and manipulating said surgical device (e.g., by tying a knot in the device) to approximate the soft tissue and hard tissue.

In yet another embodiment, a method of approximating hard tissues is contemplated, wherein a multifilament surgical device fabricated from a heterogeneous braid made from a first yarn and a second yarn in a braided construction wherein the first yarn includes at least one poly(trimethylene)terephthalate filament and the second yarns include a plurality of filaments of at least one fiber forming material is manipulated to approximate the hard tissues.

In yet other embodiments, the present disclosure relates to a surgical device that includes a suture anchor having at least one suture secured thereto, the suture containing at least one poly(trimethylene)terephthalate filament.

### BRIEF DESCRIPTION OF THE DRAWINGS

The illustrative embodiments described herein will become more readily apparent from the following description, reference being made to the accompanying drawings in which:
FIG. 1 shows a needle-suture combination in accordance with the present disclosure;
FIG. 2 is a schematic view of a heterogeneous yarn in accordance with this disclosure;
FIGS. 3A, 3B and 3C show illustrative embodiments of braids in accordance with this disclosure;
FIG. 4 is a view of an alternative embodiment of a device in accordance with the present disclosure having a spiroid braided structure; and
FIG. 5 is a view of another alternative embodiment of a device in accordance with the present disclosure having a hollow braided structure having a sheath and a core
FIG. 6 is a perspective view of a suture, suture anchor and associated suture anchor driver as in one embodiment described herein;
FIG. 7 is an enlarged area of detail of Fig. 6;
FIG. 8 is a perspective view of a two part suture anchor being assembled with sutures of the present disclosure;
FIG. 9 is a perspective view of the suture anchor of FIG. 8 being positioned on an anchor driver;
FIG. 10 is a perspective view, partially shown in section, of the suture driver being rotated to drive the suture anchor carrying sutures in accordance with the present disclosure into bone; and
FIG. 11 is a cross-sectional view partially shown in perspective of the suture anchor and associated sutures installed through tissue and into bone.

### DETAILED DESCRIPTION

Filaments in accordance with the present disclosure contain poly(trimethylene)terephthalate (referred to herein as "PTT"). PTT is a polymer having the following structure: 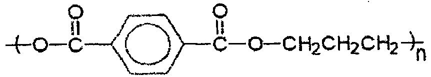 where n can be from about 2 to about 10000. In embodiments, the present filaments are made from PTT homopolymer having an average molecular weight of about 100,000 to about 1,000,000.

Filaments made from poly(trimethylene)terephthalate can be used in accordance with the present disclosure alone (e.g., as a monofilament) or to prepare yarns that can be incorporated into a braided, knitted, woven or other structure to provide a surgical device.

As used herein, the terms "fibers", "filaments" and "yarns" each may be used to construct in whole or in part a surgical suture. The term "fibers", in this context, is generally used to designate natural or synthetic structures that have a length approximately 3 orders of magnitude greater than their diameter or width. While the term "filaments" is typically used to describe "fibers" of indefinite or extreme length, and "Yarns" as a generic term for a continuous strand of twisted or untwisted "fibers" or "filaments" in a form suitable for knitting, weaving, braiding or otherwise intertwining. A plurality of yarns may be used to form a braid, knit or weave.

A "heterogeneous yarn" is a configuration containing at least two dissimilar filaments mechanically bundled together to form a yarn. The filaments are continuous and discrete, so therefore each filament extends substantially along the entire length of the yarn and maintains its individual integrity during yarn preparation, processing and use.

Unlike a heterogeneous yarn, a "homogeneous" yarn is a configuration containing substantially similar filaments. The filaments are also continuous and discrete. Therefore each filament extends substantially along the entire length of the yarn and maintains its individual integrity during yarn preparation, processing and use.

A "heterogeneous braid" is a configuration containing at least two dissimilar yarns. The two types of yarns are intertwined in a braided construction. The yarns are continuous and discrete, so therefore each yarn extends substantially along the entire length of the braid and maintains its individual integrity during braid preparation, processing and use.

This disclosure contemplates yarns that include at least one poly(trimethylene)terephthalate filament, articles made from such yarns, and use of such articles in surgery. Methods for forming fibers from poly(trimethylene)terephthalate are within the purview of those skilled in the art. The yarn can be a homogeneous yarn made entirely of filaments made from poly(trimethylene)terephthalate. In other embodiments, the yarn is a heterogeneous yarn made from at least one filament made from poly(trimethylene)terephthalate- in combination with a plurality of filaments made from at least one other fiber forming material. In embodiments, the heterogeneous yarn embodiments include a plurality of filaments made from poly(trimethylene)terephthalate in combination with a plurality of filaments made from at least one polymeric material that is not poly(trimethylene)terephthalate.

Some examples of fiber forming polymeric materials suitable for making filaments that may be used in combination with filaments made from poly(trimethylene)terephthalate include, but are not limited too, natural, synthetic, biodegradable, non-biodegradable and shape memory polymers. A particularly useful polymeric material may be selected from the group consisting of polyamides, polyesters, polyacrylonitrile, polyethylene, polypropylene, polyglycolic acid, polylactic acid, polydioxanone, poly(ε-caprolactone), polytrimethylene carbonate, and combinations of such materials.

Representative natural biodegradable fiber forming polymers include polysaccharides such as alginate, dextran, cellulose, collagen, and chemical derivatives thereof (substitutions, additions of chemical groups, for example, alkyl, alkylene, hydroxylations, oxidations, and other modifications routinely made by those skilled in the art), and proteins such as albumin, zein and copolymers and blends thereof, alone or in combination with synthetic polymers.

Representative synthetic polymer blocks include polyphosphazenes, poly(vinyl alcohols), polyamides, polyester amides, poly(amino acid)s, synthetic poly(amino acids), polyanhydrides, polycarbonates, polyacrylates, polyalkylenes, polyacrylamides, polyalkylene glycols, polyalkylene oxides, polyalkylene terephthalates, polyortho esters, polyvinyl ethers, polyvinyl esters, polyvinyl halides, polyvinylpyrrolidone, polyesters, polylactides, polyglycolides, polysiloxanes, polyurethanes and copolymers thereof.

Examples of suitable polyacrylates include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate) and poly(octadecyl acrylate).

Synthetically modified natural polymers include cellulose derivatives such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate and cellulose sulfate sodium salt. These are collectively referred to herein as "celluloses".

Representative synthetic degradable polymers include polyhydroxy acids, such as polylactides, polyglycolides and copolymers thereof; poly(hydroxybutyric acid); poly(hydroxyvaleric acid); polycarbonates; poly(amino acids); poly(hydroxyalkanoate)s, including but not limited to polyhydroxyhexanoate and polyhydroxyoctanoate; polyanhydrides; polyortho esters; poly(3-hydroxybutyric acid), poly(4-hydroxybutyric acid), poly(hydroxyvaleric acid) and blends and copolymers thereof.

Examples of non-biodegradable polymers include poly(ethylene vinyl acetate), poly(meth)acrylic acid, polyamides, polyethylene, polypropylene, polystyrene, polyvinyl chloride, polyvinylphenol, and copolymers and mixtures thereof. A further suitable non-biodegradable fiber is ultra-high molecular weight polyethylene (UHMWPE), available under the tradename SPECTRA® (Honeywell, Inc., Morristown, NJ) and Dyneema®/Dyneema® Pure (DSM High Performance Fibers B.V. Corporation, Heerlen Netherlands).

Rapidly bioerodible polymers such as poly(lactide-co-glycolide)s, polyanhydrides, and polyorthoesters, which have carboxylic groups exposed on the external surface as the smooth surface of the polymer erodes, also can be used.

Strands made from carbon fibers, steel fibers, shape memory alloy (e.g., nitinol) and other biologically acceptable inorganic fibrous materials can also be employed.

In the present invention, at least one filament made from a fiber forming material comprising polyolefin is combined with at least one filament made from PTT. In embodiments polyolefin filaments are combined with filaments made from PTT. The polyolefin is an ultra high molecular weight polyethylene. Ultra high molecular weight ("UHMW") polyethylene is a linear polymer with an average molecular weight greater than about 400,000, typically in the range of about 500,000 to about 6,000,000. UHMW polyethylene has a high tenacity and low elongation to provide articles with greatly increased strength and decreased elongation.

UHMW polyethylene typically exhibits a very substantial degree of molecular orientation (95-99%) and crystalline content (60-85%), both of which contribute to the significant strength and stability of the resulting fibers. The fibers exhibit strengths from about 375 kpsi (thousands of pounds per square inch) to about 560 kpsi, and tensile moduli of about 15 msi (millions of pounds per square inch) to about 30 msi. Ultra high molecular weight polyethylene is commercially available under the trademark SPECTRA® from Allied-Signal Technologies, Petersburg, Va., and under the trademark DYNEEMA® from DSM High Performance Fibers, JH Heerlen, The Netherlands.

The filaments made from poly(trimethylene)terephthalate may make up from about 10% to about 90% of the cross-sectional area of the heterogeneous yarns, in embodiments from about 25% to 75%, and in other embodiments from about 25% to 50% of the heterogeneous yarns.

In an embodiment of the present invention, the heterogeneous yarns are made from poly(trimethylene) terephthalate homopolymer fibers and fibers of UHMWPE with poly(trimethylene) terephthalate homopolymer fibers being about 10 to 90% of the braid.

Sutures made in accordance with the foregoing description will exhibit superior strength and resistance to abrasion, and may find particular use in medical devices includig medical devices useful in cardiac and orthopedic surgery. With respect to orthopedic surgery in particular, the suture will be useful in securing bone under high stress and abrasion as well as securing soft tissue or an implant to bone.

The filaments which form the yarns can be made using any known technique, such as, for example, melt spinning, solution spinning, gel spinning, extrusion, molding and/or solvent casting. In a preferred embodiment, the filaments can be melt spun through an extruder unit of a conventional type, such as those disclosed in U.S. Pat. Nos. 6,063,105; 6,203,564; and 6,235,869**.** The filaments of dissimilar materials can be extruded separately and subsequently brought together into a group to form a yarn, or the filaments can be extruded in a side-by-side fashion (co-extruded) and collected together to immediately form a yarn. For example, with respect to sutures, size is established according to United States Pharmacopoceia ("USP") standards. However, each yarn can contain at least ten filaments, and often more, of at least two differing material types. The filaments run parallel to each other along the length of the yarn, although twisting and commingling the filaments to form a twisted yarn is also contemplated.

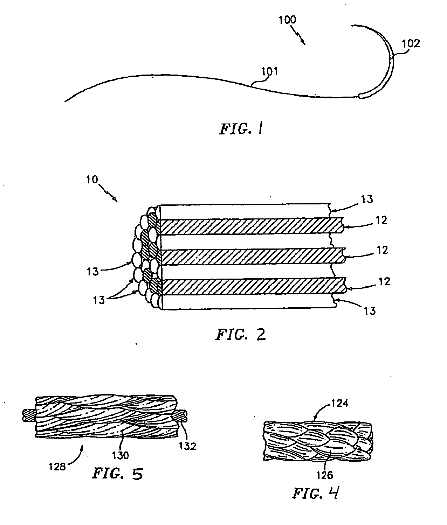

A suture 101 prepared in accordance with this disclosure can have a needle 102 attached thereto as shown in FIG. 1, to provide a needle suture combination 100. In order to facilitate needle attachment, conventional tipping agents can be applied when the suture is a multifilament suture, e.g., a braid. Two tipped ends of the suture may be desirable for attaching a needle to each end of the suture to provide a so-called double armed suture. The needle attachment can be made by any conventional method such as crimping, swaging, etc., including those described in US Pat. Nos. 5,133,738; 5,226,912; and 5,569,302 .

In one embodiment, a heterogeneous yarn 10 contains a plurality of two dissimilar filaments as shown in Figure 2. First filaments 12 are made from a first fiber forming material and second filaments 13 are made from poly(trimethylene)terephthalate. A plurality of the two dissimilar filaments are commingled to form a heterogeneous yarn.

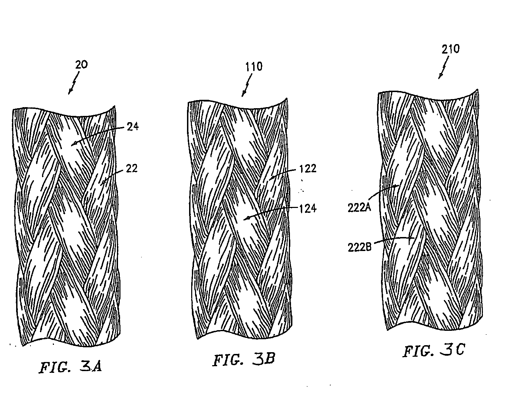

In another embodiment shown in Figure 3A, a heterogeneous braid 20 contains two dissimilar yarns. A first yarn 22 contains a plurality of filaments all made from a first fiber forming polymeric material and is thus a homogenous yarn. A second yarn 24 contains a plurality of filaments all made from poly(trimethylene)terephthalate and is likewise a homogenous yarn. The first and second homogenous yarns are intertwined to form a heterogeneous braid.

In still another embodiment shown in Figure 3B, a heterogeneous braid 110 contains a heterogeneous yarn. 122 and a homogeneous yarn 124. As described above, a heterogeneous yarn contains a plurality of two dissimilar filaments. Preferably, a first filament is made from a first fiber forming polymeric material and a second filament is made from poly(trimethylene)terephthalate. A homogeneous yarn contains a plurality of filaments made from any material capable of being spun into a filament. The heterogeneous yarn and the homogeneous yarn are intertwined to form a heterogeneous braid.

In yet another embodiment shown in Figure 3C, a braid 210 contains two similar heterogeneous yarns 222A, 222B. Each heterogeneous yarn contains a plurality of two dissimilar filaments. Preferably, a first filament is made from a first fiber forming polymeric material and a second filament is made from poly(trimethylene) terephthalate. The heterogeneous yarns are intertwined to form a braid.

Braids and/or yarns can be prepared using conventional braiding technology and equipment commonly used in the textile industry, and in the medical industry for preparing multifilament sutures. Suitable braid constructions are disclosed, for example, in U.S. Pat. Nos. 3,187,752; 3,565,077; 4,014,973; 4,043,344; 4,047,533; 5,019,093; and 5,059,213 .

In FIG. 4, there is an alternative elongated embodiment of spiroid braided construction of generally circular cross-section and comprised of heterogeneous yarns 126 combined to form a braided rope-like construction of generally circular cross-sectional configuration. Braid constructions having a circular cross-section are described in U.S. Pat. Nos. 3,565,077 and 5,019,093. In FIG. 5 there is shown a hollow braid construction 128 having a sheath constructed of heterogeneous yarns 130 and having a core 132.

Illustrative flat braided structures (suitable, e.g., for tendon repair) which can be formed using the presently described yarns include those described in U.S. Patent Nos. 4,792,336 and 5,318,575. Suitable mesh structures are shown and described, for example, in Hain et al. U.S. Patent No. 5,292,328. In addition, filaments made from poly(trimethylene)terephthalate may be incorporated into non-woven structures, such as felt. One suitable non-woven structure is shown and described in Koyfman et al. U.S. Patent No. 5,393,534.

If desired, surgical devices made using PTT filaments prepared in accordance with the disclosure can optionally be coated with one or more coating compositions to improve functional properties of the device. For example, a coating can be applied to improve surface lubricity and knot tie-down behavior. Suitable coating compositions include but are not limited to those disclosed in U.S. Pat. Nos. 3,867,190; 3,942,532; 4,047,533; 4,452,973; 4,624,256; 4,649,920; 4,716,203; 4,826,945; and 5,569,302.

The coating can be applied using any known technique such as, for example coating, dipping, spraying or other appropriate techniques.

The amount of coating composition applied to the device will vary depending upon the specific construction of the device, its size and the exact material of its construction. In general, the coating composition will constitute from about 0.5 to about 4.0 percent by weight of the coated device, or higher with a preferred range from about 1.0 percent to about 3.0 percent.

A multifilament surgical device prepared from the presently described yarns (homogeneous or heterogeneous yarns) may also be impregnated or coated with one or more medicicinal/biotherapeutic substances, e.g., those which accelerate or beneficially modify the healing process when the suture is applied to a wound or surgical site. The medically useful or therapeutic agents can include varying amounts of one or more optional ingredients, such as, for example, bioactive substances such as biocidal agents, antibiotics, antimicrobials, medicants, growth factors, anti-clotting agents, analgesic, anesthetics, anti-inflammatory, antifungals, antibacterials, etc., and the like. Medicants are defined as substances which are beneficial to the animal and tend to promote the healing process. For example, a braided suture can be provided with a therapeutic agent which will be deposited/released at the sutured site. The therapeutic agent can be chosen for its wound healing properties, capability for promoting wound repair and/or tissue growth, or for specific indications such as tissue growth. Antimicrobial agents such as broad spectrum antibiotics (gentamicin sulphate, erythromycin or derivatized glycopeptides) which are slowly released into the tissue can be applied in this manner to aid in combating clinical and sub-clinical infections in a surgical or trauma wound site. To promote wound repair and/or tissue growth, one or more biologically active materials known to achieve either or both of these objectives can also be applied. Such materials include any of several human Growth factors (HGFs), magainin, tissue or kidney plasminogen activator to cause thrombosis, superoxide dismutase to scavenge tissue-damaging free radicals, tumor necrosis factor for cancer therapy, colony stimulating factor, interferon, interleukin-2 or other lymphokines to enhance the immune system, and so forth.

A suture prepared from yarns or filaments in accordance with this disclosure can also include, for example, biologically acceptable plasticizers, antioxidants, and colorants, which can be included into the composition from which the filaments making up the yarn are made or impregnated into the heterogeneous yarns of the device.

In addition, the yarn and/or product made therefrom may be plasma treated depending upon the particular needs or intended application so as to increase the handling properties of the yarn and/or product. Techniques for plasma treating are with the purview of those skilled in the art.

As noted above PTT filaments of the present disclosure can also be utilized to form other surgical or medical articles, including, braided tapes, gauze, wound dressings, hernial repair meshes, vascular grafts (e.g. fabrics and/or tubes) anastomosis rings, prosthetic ligaments and tendons, growth matrices, drug delivery devices and other implantable medical devices.

Surgical devices prepared from PTT filaments in accordance with this disclosure can be packaged and sterilized in any conventional manner known to those skilled in the art.

Once sterilized, a device, as described herein, may be used to repair wounds or secure implants located between two or more soft tissues, two or more hard tissues, or at least one soft tissue and at least one hard tissue. For example, braided multifilament surgical device is passed through, wrapped around or secured to tissue and then the tissue is approximated by manipulating the braided multifilament surgical device, such as, for example, by tying a knot, cinching the device, applying a buckle, or the like.

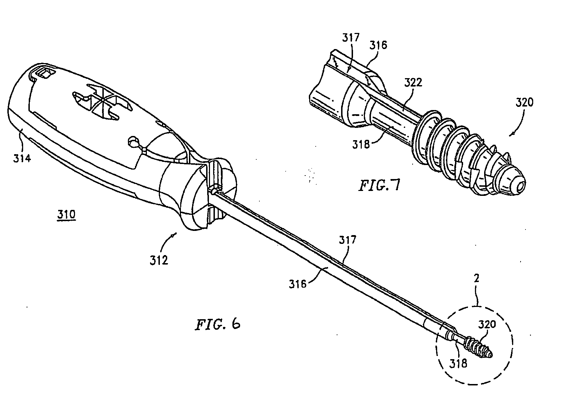

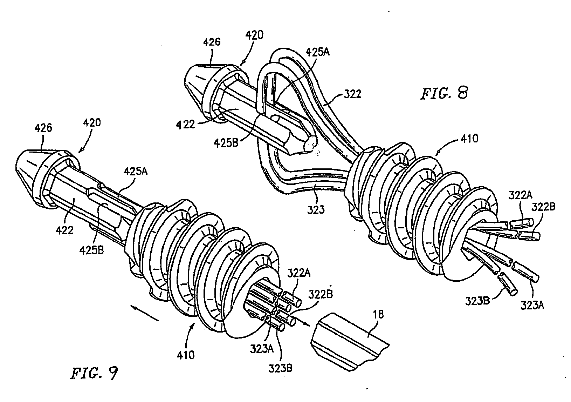

In a particularly useful embodiment, it is contemplated that the suture in accordance with the disclosure may be delivered in conjunction with a suture anchor delivery system and may be passed through tissue using an arthroscopic suturing instrument. Referring now to Figs. 6 and 7, one suitable suture anchor delivery system 310 is shown having a handle 314 with an elongate shaft 316 supporting a threaded suture anchor 320 at the distal tip 318 of the shaft 316 away from the handle 314. As shown in Fig. 7, suture 322 made in accordance with the present disclosure is attached to the suture anchor 320 and is led through trough 317 in the shaft 316 (and a corresponding trough (not shown) on the other side shaft 316) to handle 314. Referring now to Figs. 8 and 9, one method of pre-attaching a pair of sutures 322, 323 to a suture anchor is shown. As shown, suture anchor 320 consists of two parts, a hollow-threaded body portion 410 and a tip portion 420 having a shaft 422 insertable into the hollow body portion 410 and configured to receive and hold two sutures 322; 323 through transverse apertures 425A, 425B, as shown. Enlarged tip bead section 426 does not pass into or through the hollow threaded body 410, thereby retaining the suture relative to the suture anchor as the sutures 322, 323 are placed under tension by pulling on proximal ends 322A, 322B, 323A, 323B. Of course, numerous other types of suture anchors and methods of attaching sutures and in accordance with the present disclosure will occur to those skilled in the art. By way of example, the suture alternatively may be attached to a push-in-type anchor rather than a screw-in type anchor. See for example, Larsen U.S. Patent No. 5,993,459. Preferably, the proximal ends of the suture are attached to needles (not shown) suitable for use during surgery to pass the suture through tissue and, via appropriate manipulation (e.g., knot tying and/or cinching) secure the tissue relative to the anchor.

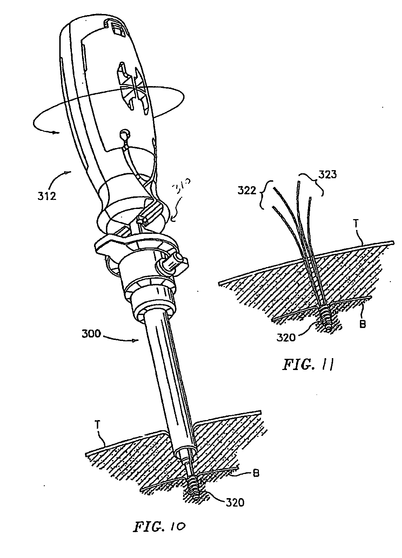

In use during an arthroscopic procedure a cannula 300 is inserted into the joint capsule and the shaft 316 of the suture anchor delivery system 310 is inserted through the cannula 300 to a prepared site suitable to receive a suture anchor 320. Fig. 10 shows shaft 316 of delivery system 310 inserted through cannula 300 with suture anchor 320 inserted into bone B. Fig. 11 shows the suture anchor 320 released from the delivery system 310 leaving the sutures 322, 323 available for manipulation to secure the soft tissue T to bone B. In a further preferred embodiment the sutures are attached to needles (not shown) suitable for passing through soft tissue. The needles may be traditional suture needles suitable for use with an arthroscopic suturing instrument. One such instrument is the Arthrosew® instrument (Covidien, North Haven, CT) which utilizes a double ended surgical incision mender. Most preferably, the handle portion of the suture anchor delivery system includes releasable suture management members (not shown) which hold the suture needles for use. If the suture needles are to be used with a suturing device, the suture management members are configured to engage the suturing instrument in a suitable manner to transfer control of the needle to the suturing instrument. The needles and suture(s) are passed through soft tissue and the suture is manipulated, such as by forming in the suture, to secure the soft tissue relative to the suture anchor. Thereafter, the patient is closed in a suitable manner depending upon whether the procedure was conducted as an open, mini-open or closed arthroscopic approach.

In the context of a suture anchor, a suture constructed in accordance with the present disclosure provides significantly enhanced resistance to abrasion as the suture is manipulated, including drawing the suture through the suture eyelets of the suture anchor, forming knots in the suture, and cinching the knots down tightly for secure approximation of the soft tissue to bone.

Various modifications and variations of the filaments, yarns, braids and devices and uses thereof will be apparent to those skilled in the art from the foregoing detailed description. Such modifications and variations are intended to come within the scope of the following claims.

## Claims

1. A multifilament surgical device comprising a heterogeneous yarn including at least one first filament made from poly(trimethylene)terephthalate and at least one second filament made from a fiber forming material comprising ultra high molecular weight polyethylene (UHMWPE).

2. The multifilament surgical device of claim 1 which is selected from the group consisting of a suture, mesh and tape.

3. A sterile braid comprising a heterogeneous yarn in accordance with claim 1.

## Patentansprüche

1. Mehrfädige chirurgische Vorrichtung, umfassend ein heterogenes Garn, das mindestens einen ersten Faden aufweist, der aus Poly(trimethylen)terephtalat hergestellt ist, und mindestens einen zweiten Faden, der aus einem faserbildenden Material hergestellt ist, umfassend Polyethylen mit ultrahohem Molekulargewicht (UHMWPE).

2. Mehrfädige chirurgische Vorrichtung nach Anspruch 1, die aus der Gruppe ausgewählt ist, bestehend aus einer Naht, einem Netz und einem Band.

3. Steriles Geflecht, umfassend ein heterogenes Garn nach Anspruch 1.

## Revendications

1. Dispositif chirurgical multifilamentaire comprenant un fil hétérogène comprenant au moins un premier filament constitué de poly(téréphtalate de triméthylène) et au moins un second filament constitué d'un matériau fibrogène comprenant du polyéthylène de poids moléculaire ultra élevé (UHMWPE).

2. Dispositif chirurgical multifilamentaire selon la revendication 1, qui est choisi dans le groupe constitué d'une suture, d'une maille et d'un ruban.

3. Tresse stérile comprenant un fil hétérogène selon la revendication 1.
